(19) Europäisches Patentamt
European Patent Office
Office européen des brevets

(11) **EP 4 644 953 A1**

(12) **EUROPEAN PATENT APPLICATION**
published in accordance with Art. 153(4) EPC

(43) Date of publication:
**05.11.2025 Bulletin 2025/45**

(21) Application number: **23911536.3**

(22) Date of filing: **01.12.2023**

(51) International Patent Classification (IPC):
*G01T 1/172* *(2006.01)*   *A61N 5/10* *(2006.01)*
*G01T 1/17* *(2006.01)*   *G01T 1/20* *(2006.01)*
*G01T 1/24* *(2006.01)*   *G01T 1/161* *(2006.01)*

(52) Cooperative Patent Classification (CPC):
**A61N 5/10; G01T 1/161; G01T 1/17; G01T 1/172;
G01T 1/20; G01T 1/24**

(86) International application number:
**PCT/JP2023/043056**

(87) International publication number:
**WO 2024/142755 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.12.2022 JP 2022210091**

(71) Applicant: **The University of Osaka
Suita-shi, Osaka 565-0871 (JP)**

(72) Inventors:
• **MURATA Isao
  Suita-shi, Osaka 565-0871 (JP)**
• **SATO Fuminobu
  Suita-shi, Osaka 565-0871 (JP)**
• **TAMAKI Shingo
  Suita-shi, Osaka 565-0871 (JP)**
• **KUSAKA Sachie
  Suita-shi, Osaka 565-0871 (JP)**

(74) Representative: **Vossius & Partner
Patentanwälte Rechtsanwälte mbB
Siebertstrasse 3
81675 München (DE)**

(54) **MEASURING DEVICE AND MEASURING METHOD FOR MEASURING EFFECTIVENESS OF BORON NEUTRON CAPTURE THERAPY**

(57)    To provide a BNCT-SPECT technology capable of measuring prompt Gamma rays generated by a BNC reaction in real time with excellent accuracy in BNCT, and the measurement device measuring the therapeutic effect of boron neutron capture therapy, comprises a collimator having holes of a predetermined shape formed at equal intervals vertically and horizontally, a detector arranged at the deepest part of each hole for detecting prompt Gamma rays generated by neutron irradiation of boron, and a calculation processing unit which, when a gamma ray is detected by the detector, performs coincidence counting or non-coincidence counting to create a PHS separately for cases in which the gamma ray is not detected by other surrounding detectors at the same time and cases in which gamma rays are detected by other surrounding detectors at the same time, and displays the overall gamma ray intensity distribution in three dimensionally.

FIG. 1

**Description**

[TECHNICAL FIELD]

The present invention relates to a measurement device for measuring the effect of boron neutron capture therapy, and a measurement method for measuring the effect of boron neutron capture therapy using the measurement device.

[BACKGROUND ART]

In recent years, research into the practical application of boron neutron capture therapy (BNCT) as a new next-generation cancer treatment method has accelerated (see, for example, Patent Document 1), and its widespread use around the world is desired.

This BNCT is a radiation therapy method in which boron ($^{10}B$) is selectively accumulated in tumor cells (cancer cells) and then neutrons are irradiated from outside. As a result of a nuclear reaction with neutrons (mainly epithermal neutrons), the charged particles (a particles and Li atomic nuclei) released from boron have a range that is approximately the same as that of cells, and so BNCT has attracted attention as a treatment method that can selectively kill only cancer cells without damaging normal cells.

However, there are some unsolved problems in the widespread use of BNCT. It is known that one of the problems is that the therapeutic effect cannot be known in real time during the treatment (neutron irradiation).

To solve this problem, the Japanese Society for Neutron Capture Therapy has proposed a SPECT (Single Photon Emission Computed Tomography) device (BNCT-SPECT) that measures 478 keV gamma rays emitted from Li nuclei released in the neutron reaction of boron, converts them into three-dimensional images, and performs on-site observation.

[PRIOR ART DOCUMENTS]

[PATENT DOCUMENT]

[Patent Document 1] WO2018/181395 publication

[SUMMARY OF INVENTION]

[PROBLEM TO BE SOLVED BY THE INVENTION]

However, BNCT-SPECT is a measurement performed during a medical procedure, and unlike normal diagnostic SPECT, there are many restrictions on the measurement of physical quantities, making it difficult and not yet in practical use.

That is, in the case of normal diagnostic SPECT, since only the radiation source for measurement is used, there is no background noise, and the S/N (signal/noise) ratio during measurement is increased, enabling measurements to be performed with excellent accuracy.

In contrast, in the case of BNCT-SPECT, instead of measuring neutrons irradiated as primary radiation for treatment, it is necessary to measure prompt Gamma rays generated as secondary radiation by the BNC reaction ($^{10}B$ (n, a) $^{7}Li$) in real time. However, since the intensity of these prompt Gamma rays is orders of magnitude smaller than the primary radiation (neutrons) that becomes background (noise), it is not easy to measure them with high accuracy in real time to know the effectiveness of treatment.

In addition, with BNCT, there are various constraints, such as the need to irradiate neutrons from close to the patient, the need for various devices and instruments to be attached to around the tumor and the patient, and the unavoidable change in patient position during treatment. Therefore, unlike X-ray CT, MRI, and regular SPECT diagnosis, the SPECT equipment cannot be moved 360 degrees, and from this perspective, it is not easy to measure with high accuracy in real time and know the effectiveness of treatment.

Therefore, an object of the present invention is to provide a BNCT-SPECT technique capable of measuring prompt Gamma rays generated by a BNC reaction in real time with excellent accuracy in BNCT.

[MEANS FOR SOLVING THE PROBLEM]

The present inventors have conducted extensive studies for solving the above-mentioned problems, and have found that the above-mentioned problems can be solved by the invention described below, thereby completing the present invention.

The invention describes in claim 1 is

a measuring device for measuring a therapeutic effect in boron neutron capture therapy, which kills tumor cells by irradiating neutrons to boron that has been accumulated in tumor cells in advance, comprising:

a collimator having holes of a predetermined shape formed vertically and horizontally at equal intervals;
a detector arranged at the deepest portion of each of the holes for detecting prompt Gamma rays emitted by the irradiation of the boron with the neutrons; and
a calculation processing unit that displays the entire gamma ray intensity distribution three-dimensionally characterized in,
when a gamma ray is detected by the detector,

(1) if no gamma ray is detected by other detectors in the vicinity at the same time, a pulse height distribution (hereinafter referred to as "PHS") is created based on the single detected gamma ray,
(2) if a gamma ray is detected by other detectors in the vicinity at the same time,
(2-a) if the total number of gamma rays detected is three or more, all signals are discarded and no PHS is created (non-coincidence counting),
(2-b) if the total number of gamma rays detected is two,
(2-b-a) if the sum of the two pulse heights is within a predetermined peak range, the two signals are discarded and the photoelectric peak count corresponding to the predetermined peak is increased by one (coincidence counting);
(2-b-b) if the sum of the two pulse heights is not within a predetermined peak range, the two signals are discarded (non-coincidence counting); and

based on the counts accumulated in each detector within a given time, the overall gamma ray intensity distribution is displayed in three dimensions.

*(Please note the following statement is not described in the original Japanese description. But to clarify the meaning of "the predetermined peak range" the following statement is added here:*
*" The predetermined peak range means a range in which the signal has energy equivalent to 478 keV. The range equivalent to 478 keV is preferably set to the range of the energy resolution of the detector. The meaning of this predetermined peak range is the same for the inventions described in claims 10 and 12 below.")*

The invention described in claim 2 is;

the measuring device according to claim 1, wherein
an incidence order determination mechanism that determines an incidence order of the gamma rays into the detector is added to the calculation processing unit, and
the device is configured to perform coincidence counting processing only when the detector into which the γ-ray first entered is specified.

The invention described in claim 3 is

the measurement device according to claim 1 or 2, characterized in that;
a detector for detecting transmitted gamma rays and having a size large enough to cover the entire detector is installed on the rear side of the detector, and
the device is configured to perform
when gamma rays are detected simultaneously by the transmitted gamma ray detector and one of the detectors,

(1) when the sum of the pulse heights of the two detected gamma rays is within a predetermined peak range of the PHS, the two detected signals are discarded, and considered to have been detected by the detector, and the photoelectric peak count is increased by one (coincidence counting);
(2) when the sum of the pulse heights is not within a predetermined peak range, the two detected signals are discarded.

The invention described in claim 4 is
the measuring device according to claim 1 or 2, wherein the collimator is made of tungsten.
The invention described in claim 5 is
the measuring device according to claim 1 or 2, wherein the hole of the collimator is formed in a square tube shape or a cylindrical shape.

The invention described in claim 6 is
the measuring device according to claim 5, wherein the collimator hole is formed in a square tube shape with one side of 3 to 5 mm or a cylindrical shape with a diameter of 3 to 5 mm.

The invention described in claim 7 is
the measuring device according to claim 5, wherein the length of the hole of the collimator is 20 to 35 cm.

The invention described in claim 8 is
the measuring device according to claim 1 or 2, wherein the detector is a semiconductor detector or a scintillator.

The invention described in claim 9 is
the measuring device according to claim 8, wherein the detector is a GAGG(Ce) scintillator.

The invention described in claim 10 is
a method for measuring a therapeutic effect in boron neutron capture therapy using the measurement device according to claim 1, comprising:

a detection step of detecting a gamma ray, which is emitted promptly by a nuclear reaction with boron due to irradiation with neutrons and then enters a hole in the collimator, by the detector;
a calculation processing step wherein,
if a gamma ray is detected in the detection step,

(1) if no gamma ray is detected by other detectors in the vicinity at the same time, a PHS is created based on the single detected gamma ray,
(2) if a gamma ray is detected by other detectors in the vicinity at the same time,
(2-a) if the total number of gamma rays detected is three or more, all signals are discarded and no PHS is created (non-coincidence counting),
(2-b) if the total number of gamma rays detected is two,
(2-b-a) if the sum of the two pulse heights is within a predetermined peak range, the two signals are discarded and the photoelectric peak count corresponding to the predetermined peak is increased by one (coincidence counting),
(2-b-b) if the sum of the two pulse heights is not within a predetermined peak range, the two signals are discarded (non-coincidence counting); and

a display step of creating and displaying a three-dimensional intensity distribution of the entire gamma rays based on the counts accumulated in each detector within a given time.

The invention described in claim 11 is

the measurement method according to claim 10, wherein
a coincidence counting process is performed in the calculation processing step by using the measurement device according to claim 2;
an order of incidence of the gamma rays on the detector is determined; and then a coincidence counting process is performed only on the gamma ray determined to have been incident first on the detector.

The invention described in claim 12 is

the measurement method according to claim 10 or 11, characterized in that
a gamma ray is detected by a transmitted gamma ray detector installed on the rear side of the detector, using the measurement device according to claim 3;
when gamma rays are detected simultaneously by the transmitted gamma ray detector and one of the detectors,

(1) when the sum of the pulse heights of the two detected gamma ray signals is within the predetermined pulse height range, the two detected signals are discarded, and the photoelectric peak count corresponding to the predetermined peak is increased by one (coincidence counting);
(2) if the sum of the two pulse heights is not within the predetermined pulse height range, the two detected signals are discarded (non-coincidence counting).

The invention described in claim 13 is
the measurement method according to claim 10, wherein an accelerator BNCT is used as the neutron irradiation source.

**EP 4 644 953 A1**

[EFFECT OF THE INVENTION]

According to the present invention, it is possible to provide a BNCT-SPECT technique capable of measuring prompt Gamma rays generated by a BNC reaction in real time with excellent accuracy in BNCT.

[BRIEF DESCRIPTION OF THE DRAWINGS]

FIG. 1 is a schematic diagram illustrating the principle of BNCT-SPECT.
FIG. 2 is a diagram illustrating the Compton effect and the photoelectric effect in BNCT-SPECT.
FIG. 3 is a diagram showing an example of PHS obtained by one detector (scintillator) in BNCT-SPECT.
FIG. 4 is a diagram showing an example of a PHS actual measured in BNCT-SPECT.
FIG. 5 is a diagram for explaining coincidence counting in BNCT-SPECT.
FIG. 6 is a diagram for explaining non-coincidence counting in BNCT-SPECT.
FIG. 7 is a diagram showing the relationship between the increase/decrease in the electron energy $E_{el}$ of a Compton electron $E_{1c}$ and the energy $E_{hv'}$ of a photoelectron $E_{2c}$ during Compton scattering and the scattering angle.
FIG. 8 is a diagram (a) for explaining the state in which a transmission gamma ray detector (scintillator) is installed in one embodiment of the present invention, and a diagram (b) for explaining the path of a prompt Gamma ray.

[EMBODIMENTS FOR CARRYING OUT THE INVENTION]

[1] Basic concept of the present invention

Prior to describing specific embodiments of the present invention, the basic concept (mechanism) of the present invention will be described.

1. Principle of BNCT-SPECT

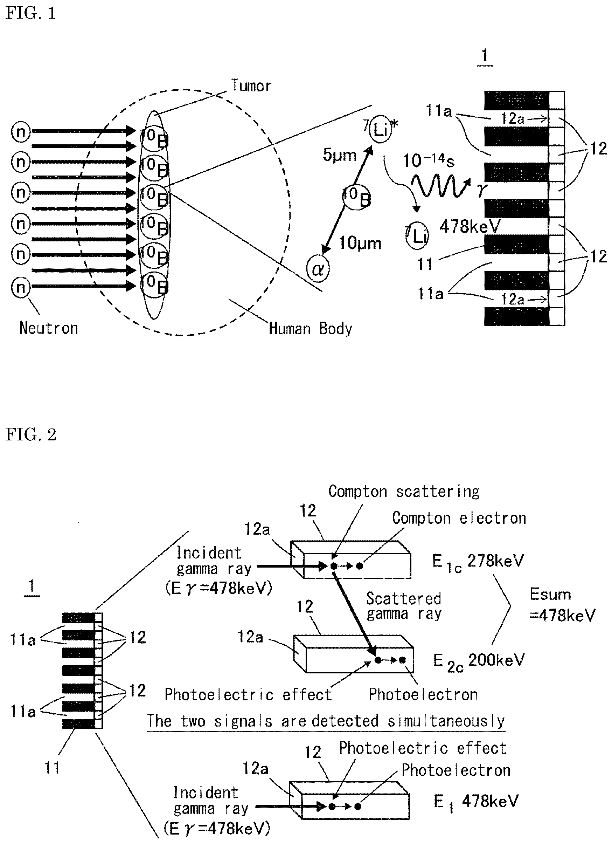

FIG. 1 is a schematic diagram for explaining the principle of BNCT-SPECT. In FIG. 1, 1 is a BNCT-SPECT, 11 is a collimator, and 12 is a detector (scintillator). Holes 11a of a predetermined shape are formed vertically and horizontally at equal intervals in the collimator 11, and a detector (scintillator) 12 is arranged at the deepest part of the holes. A photomultiplier tube or an MPPC (Multi-Pixel Photon Counter), not shown, is coupled to the detector (scintillator) 12.
As shown in FIG. 1, boron ($^{10}$B) is accumulated in tumors (cancer cells) inside the human body by administering drugs containing boron ($^{10}$B), such as BPA (Boronophenylalanine) or BSH (Disodium Mercaptoundecahydro-decaborate), intravenously.
Thereafter, when neutrons n are irradiated from an external neutron supply unit (not shown) toward the boron ($^{10}$B) accumulated in the tumor cells, the following BNC reaction ($^{10}$B (n, $\alpha$) $^7$Li) is induced.

$$^{10}B + n \rightarrow {}^7Li + \alpha + 2.79 \text{ MeV (6.1\%)}$$

$$^7Li* + \alpha + 2.31 \text{ MeV (93.9\%)}$$

The energy of the charged particles generated at this time [E$\alpha$ = 1.47 MeV, Eli = 0.84 MeV (93.9% branching)] kills the tumor cells, but the range of these charged particles is about 10 $\mu$m, which is almost the same as the size of a human cell as mentioned above, and there is little possibility of damaging normal cells adjacent to the tumor cells, so it is said to be the only radiation therapy that is very sharp compared to other radiation therapies and has tumor cell selectivity. Note that neutrons only have an effect that is said to not kill normal cells compared to charged particles.
The Li produced with a branching ratio of 93.9% is in an excited state ($^7$Li*) and transitions promptly (in about $10^{-14}$ seconds) to the ground state $^7$Li, emitting a 478 keV gamma ray (prompt Gamma ray).
The intensity of this prompt Gamma ray is the number of BNC reactions, and it is clear the number is proportional to the local boron dose accumulated in the tumor. Therefore, if the intensity distribution (PHS) of the prompt Gamma ray can be measured three-dimensionally using a SPECT device, the number of BNC reactions occurring in tumor cells can be estimated.
Since the number of occurrences of this BNC reaction is a parameter directly proportional to the therapeutic effect, it becomes possible to know the therapeutic effect in real time with excellent accuracy.

2. Specific BNCT-SPECT techniques

Next, a specific BNCT-SPECT technique will be described.

As shown in FIG. 1, in the BNCT-SPECT 1, one detector (scintillator) 12 is arranged at the deepest part of each of the holes 11a of the collimator 11. This collimator 11 parallelizes and converges gamma rays generated by neutron irradiation, thereby detecting multiple secondary gamma rays generated by the Compton effect and the photoelectric effect from a gamma ray incident on one detector (scintillator) 12, but Compton scattered gamma rays may also be detected simultaneously by multiple adjacent detectors (scintillators) (crosstalk phenomenon).

FIG. 2 is a diagram for explaining the Compton effect and the photoelectric effect in the above-mentioned BNCT-SPECT.

(a) Compton effect

As shown in the example in the upper right corner of FIG. 2, a primary gamma ray (Ey = 478 keV) incident on the surface 12a of one detector (scintillator) 12 collides with an electron inside the detector (scintillator) 12, producing Compton electrons $E_{1c}$ (energy = 278 keV) and also producing scattered gamma rays (Compton scattering).

The scattered gamma rays then enter an adjacent detector (scintillator) 12, and generate photoelectrons $E_{2c}$ (energy=200 keV) due to the photoelectric effect. The energy of the photoelectrons $E_{2c}$ corresponds to the energy ($E_{hv'}$) of the scattered gamma rays described below.

Since the Compton electron $E_{1c}$ and the photoelectron $E_{2c}$ move at the speed of light, they are detected simultaneously. The total energy $E_{sum}$ is 478 (= 278 + 200) keV, which is equal to the energy Ey of the incident gamma ray of 478 keV.

(b) Photoelectric effect

On the other hand, as shown in the lower right part of FIG. 2, a gamma ray (Ey = 478 keV) incident on the surface 12a of one detector (scintillator) 12 can produce a photoelectron $E_1$ (energy = 478 keV) due to the photoelectric effect without scattering with electrons inside the detector (scintillator) 12.

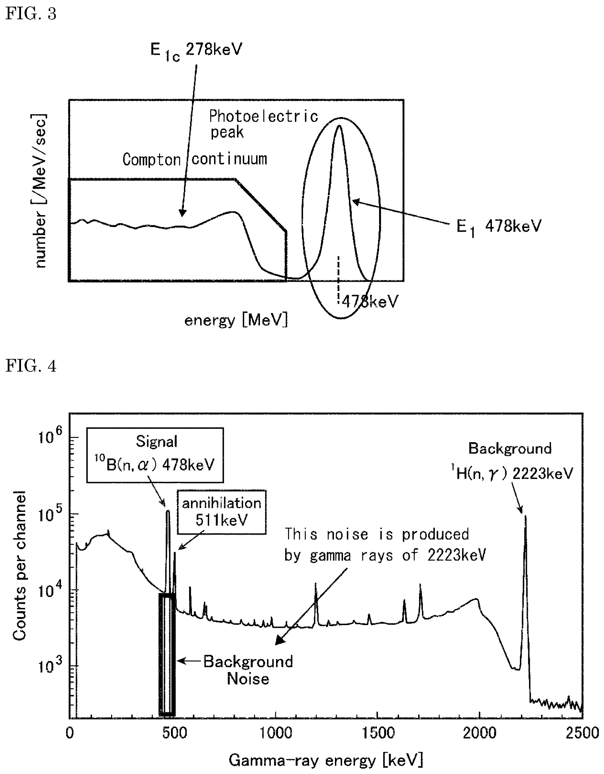

FIG. 3 is a diagram showing an example of PHS obtained by one detector (scintillator) based on the above. In FIG. 3, the Compton continuum extends up to 478 keV, and a photoelectric peak is obtained at 478 keV. In this case, since what is necessary to confirm the therapeutic effect is the photoelectric peak at 478 keV, it can be said that the above-mentioned $E_{1c}$ and $E_{2c}$ are merely noise.

3. Difficulties in actual measurement

However, as shown in FIG. 4, the actual measured PHS contains a lot of background noise near the 478 keV peak, such as the Compton continuum of the 2223 keV gamma ray emitted by the $^1$H (n, y) reaction, electron pair production, and the 511 keV annihilation gamma ray generated by β+ decay. Therefore, it is very difficult to separate these background noises and selectively measure only the 478 keV gamma ray to obtain the absolute value and spatial distribution of the dose with high accuracy. The $^1$H (n, y) reaction occurs due to the reaction between the water in the human body and the neutron beam.

The PHS shown in FIG. 4 is formed by combining the signal count number (Net) due to 478 keV gamma rays and the background noise count number (BG). Therefore, to obtain the absolute value and spatial distribution of the dose with excellent accuracy, it is necessary to reduce the background noise count number (BG) as much as possible by using a detector that is small but has high detection efficiency and appropriate energy resolution.

Specifically, since it is known that the measurement accuracy (accuracy) is expressed by the following (Formula 1), if Net is made as large as possible while BG is made as small as possible, it can be seen that the calculation result of (Formula 1) will be a small value, and excellent accuracy will be obtained.

[Formula 1]

$$\text{Measurement accuracy} = \frac{\sqrt{BG \cdot 2 + Net}}{Net} \qquad \text{(Formula 1)}$$

4. Solution in the present invention

(1) Simultaneous use of coincidence and non-coincidence counting methods

The inventors considered that, since the peak to be measured in the obtained PHS in the signals (in the case of two signals, $E_{1c}$ and $E_{2c}$) measured simultaneously in the above-mentioned crosstalk phenomenon is determined to be 478 keV as described above, it would be possible to improve accuracy by appropriately applying the coincidence counting method and the non-coincidence counting method depending on the measured peak and eliminating unnecessary signals.

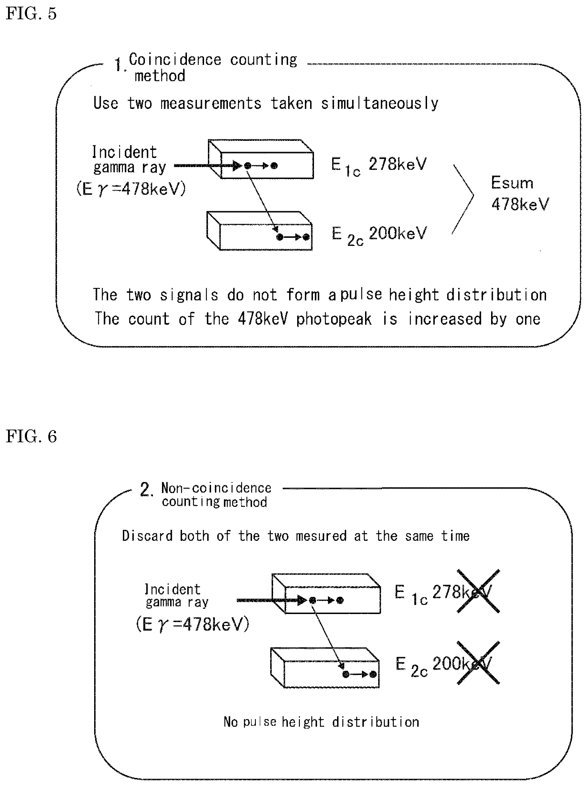

Specifically, when $E_{sum}$ is within the range of a predetermined peak (here, 478 keV) (for example, 35 keV of full width at half maximum FWHM, but other numerical ranges may be adopted as necessary), instead of discarding both, the count of the 478 keV photopeak is increased by one (coincidence counting process) as shown in FIG. 5.

If $E_{sum}$ is outside the predetermined peak range, both are discarded and no PHS is made, as shown in FIG. 6 (non-coincidence counting process).

Specifically, when gamma rays are detected by the detector,

(1) if no gamma ray is detected by other detectors in the vicinity at the same time, a PHS is created based on the single detected gamma ray,
(2) if a gamma ray is detected by other detectors in the vicinity at the same time,
(2-a) if the total number of gamma rays detected is three or more, all signals are discarded and no PHS is created (non-coincidence counting),
(2-b) if the total number of gamma rays detected is two,
(2-b-a) if the sum of the two pulse heights is within a predetermined peak range, the two signals are discarded and the photoelectric peak count is increased by one (coincidence counting);
(2-b-b) if the sum of the two pulse heights is not within a predetermined peak range, the two signals are discarded (non-coincidence counting).

In this way, by simultaneously using coincidence counting and non-coincidence counting in the analysis of the actual measured PHS, it is possible to increase the photoelectric peak count while decreasing the Compton continuum count. As a result, it is possible to increase the Net count while decreasing the BG count, thereby improving accuracy.

(2) Determination of the incident element during coincidence counting

In the present invention, in the coincidence counting process, to improve accuracy, it is necessary to know which detector (scintillator) the signal first enters.

That is, as described above, coincidence counting is a signal processing in which the peak values of two signals ($E_{1c}$, $E_{2c}$) are summed when they are simultaneously incident on two detectors (scintillators), but in SPECT, it is necessary to know which detector (scintillator) the signal first incidents on, because it indicates the detection location. That is, when $E_{2c} + E_{1c} = 478$ keV, the signal is the signal to be measured, but it is necessary to know which detector the signal first incidents on.

However, because the two signals are measured simultaneously, although it is technically possible to determine which one was incident first, that is, to identify the $E_{1c}$ detector (scintillator), this is nearly impossible in practice.

Therefore, the present inventors focused on the scattering angle during Compton scattering. That is, the present inventors noticed that the increase and decrease in the photoelectron energy $E_{e1}$ and the energy $E_{hv'}$ of the scattered gamma ray during Compton scattering have a specific relationship with the scattering angle.

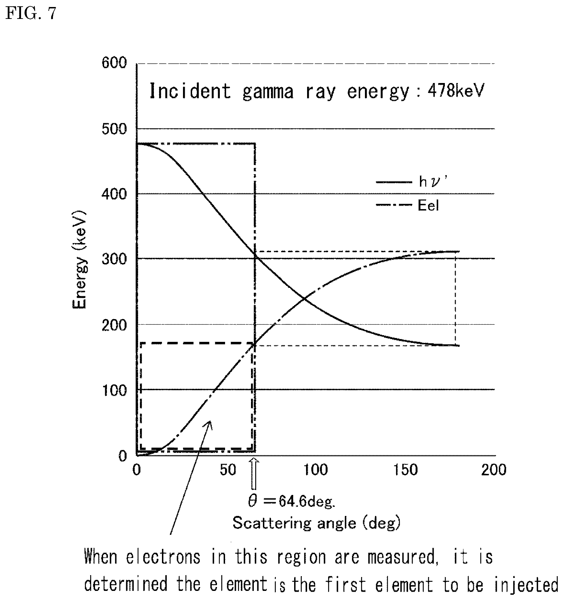

FIG. 7 is a graph showing the relationship between the increase/decrease in the energy $E_{el}$ of the photoelectrons and the energy $E_{hv'}$ of the scattered gamma rays during the Compton scattering described above and the scattering angle, and is a graph obtained by plotting $E_{el}$ and $E_{hv'}$ against the scattering angle $\theta$ (degrees).

From Fig. 7, it is seen that in the region up to a scattering angle of 64.6 degrees, $E_{hv'}$ is always larger than $E_{el}$, and $E_{el}$ and $E_{hv'}$ can be clearly distinguished. In addition, a detector (scintillator) that measures electrons (photoelectrons) in the region enclosed by a thick dashed square (<166.5 keV) detects $E_{el}$, and therefore can be determined to be the detector (scintillator) into which a gamma ray was first incident. On the other hand, at angles greater than 64.6 degrees, for example, even if an electron with an energy of 200 keV is measured, it is not possible to determine whether it is $E_{el}$ or $E_{hv'}$, and it is not possible to determine which one was incident first.

By integrating the Klein-Nishina theoretical formula, it was found that the probability of scattering occurring in this region is 57%. Therefore, by combining this knowledge with the above-mentioned coincidence counting method, it is possible to further improve the accuracy.

(3) Installation of a detector for detecting transmitted radiation (scintillator)

The simultaneous use of the above-mentioned coincidence and non-coincidence counting methods is basically performed by utilizing the signals of the other detectors (scintillators) except for the one detector (scintillator) into which the gamma rays are incident. However, after the amma rays are scattered by the detector (scintillator), the scattered gamma rays may penetrate the detector as they are, which may cause a deterioration in accuracy.

The inventors came up with the idea that to detect these transmitted gamma rays, it would be sufficient to install a separate detector (scintillator) large enough to cover all the detectors (scintillators) behind the detectors (scintillators) as a detector (scintillator) for detecting transmitted gamma rays. They the inventors confirmed that the accuracy was further improved when the above-mentioned coincidence and non-coincidence counting methods were used simultaneously.

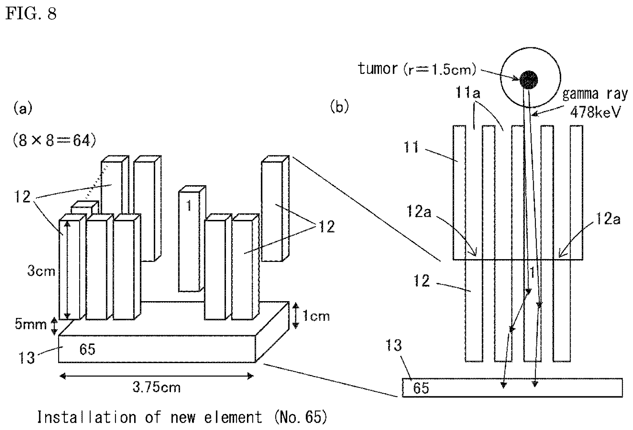

FIG. 8 (a) is a diagram for explaining the state in which an additional transmission gamma ray detector (scintillator) 13 is installed, and FIG. 8 (b) is a diagram for explaining the path of a prompt Gamma ray. In FIG. 8 (a), the 65th transmission gamma ray detector (scintillator) 13 is installed on the back side of the 8 × 8 = 64 detectors (scintillators) 12, 12, 12, ...

In this case, as shown in FIG. 8 (b), the detector (scintillator) 13 for detecting transmitted gamma rays detects only gamma rays that have passed through the front detectors (scintillators) 12, 12, 12, ... That is, when a gamma ray is detected by the detector 13 for detecting transmitted gamma rays, if there is no detector that has been counted simultaneously, the signal is discarded, and if there is a detector that has been counted simultaneously, since it is clear that the gamma ray entered the detector before the detector 13 for detecting transmitted gamma rays, the detector is the incident detector. In fact, since there are many single scattering events as shown in FIG. 8 (b), it is possible to improve accuracy by handling it in this way. Note that there are few multiple scattering events as shown in the same figure, which are two or more times, but in this case, the signal is discarded. This is because in the case of multiple scattering, it is unclear which detector entered first.

[2] Specific embodiments

Next, the present invention will be described in detail based on specific embodiments.

1. Fabrication of BNCT-SPECT

First, the BNCT-SPECT 1 shown in Fig. 1 is fabricated. In this embodiment, taking into consideration that the processing time of a normal BNCT is less than one hour, the design goal of the BNCT-SPECT is to obtain measurement results with an accuracy of less than 5% and a spatial resolution of less than 5 mm in approximately 60 minutes of measurement.

The above-mentioned accuracy is defined as a statistical accuracy, which is closely related to the performance of the detector (scintillator) used, and is calculated rigorously by the Monte Carlo code MCNP5.

On the other hand, spatial resolution is defined as the diameter of the observation area in a detector (scintillator) located away from the center of the tumor, and is closely related to the performance of the collimator, and is rigorously calculated by the Monte Carlo code MCNP5.

(1) Collimator

The collimator 11 is made of tungsten and has 8 × 8 = 64 holes 11a with a diameter of 3.5 mm and a length of 26 cm at a hole pitch of 4.0 mm (size: 3.75 cm × 3.75 cm × 26 cm). In addition to tungsten, the collimator 11 may be made of lead or heavy metal as needed. The number of holes 11a can be changed as needed. The shape of the holes 11a is not particularly limited, but a square tube shape with a side of 3 to 5 mm or a cylindrical shape with a diameter of 3 to 5 mm is preferable, and the length is preferably 20 to 35 cm.

(2) Detector (scintillator)

As the detectors (scintillators) 12, GAGG(Ce) scintillators (gadolinium aluminum gallium garnet: $Gd_3Al_2Ga_3O_{12}$ (Ce)) of 3.5 × 3.5 × 30 mm are placed one by one (64 in total) in the deepest part of each of the holes 11a of the collimator 11. Then, as shown in FIG. 8, a GAGG(Ce) scintillator of 3.75 cm × 3.75 cm × 1 cm is placed as the 65th transmitted gamma ray detection detector (scintillator) 13 on the back side of the 64 detectors (scintillators) 12 with an interval of 5 mm.

The detector is not particularly limited as long as it can detect prompt Gamma rays, and may be a semiconductor detector or a scintillator. However, it is preferable to use a GAGG(Ce) scintillator in consideration of the following: the density is as high as 6.63 $g/cm^3$ and thus has high detection efficiency; the light emission amount is as high as 60,000 photons/MeV and thus has sufficient energy resolution; it is not deliquescent or hygroscopic and can be used even under high humidity conditions, so it is easy to handle; the melting point is high and has excellent temperature stability; and it can maintain a high light emission amount even under high temperatures.

2. Treatment with BNCT

Next, a neutron irradiation port is placed in close contact with the patient's tumor is placed in close contact with the wall from which neutrons are emitted, and neutrons are irradiated toward the affected area. Note that boron is accumulated in the tumor area of the patient in advance, as described above.

This induces the BNC reaction as described above, and the treatment progresses by killing the tumor cells, and prompt Gamma rays are emitted.

3. Measurements using BNCT-SPECT

At the same time, a BNCT-SPECT is placed in close contact with the patient's tumor on the opposite side of the wall, and shooting is performed in the range of 90 degrees to 180 degrees. Note that it is preferable to avoid placing the BNCT-SPECT at an angle of 0 degrees with respect to the irradiation direction of the neutron beam, so that the detector (scintillator) is not damaged by the transmitted neutron beam.

The PHS obtained by the shooting is processed using the simultaneous use of the coincidence and non-coincidence counting methods described above to create a three-dimensional image of 478 keV gamma rays, and the accuracy is calculated based on the above-mentioned Formula 1.

As mentioned above, the obtained three-dimensional image shows the intensity distribution of prompt Gamma rays, and since the intensity is proportional to the dose to boron, the therapeutic effect of BNCT can be easily confirmed by analyzing this image in real time.

When the present inventors performed calculation evaluation according to the above-described embodiment, it was confirmed that a spatial resolution of 5.1 mm and a statistical accuracy of 4.4% were obtained, which are close to the initial target values.

Although the present invention has been described above based on the embodiment, the present invention is not limited to the above embodiment. Various modifications can be made to the above embodiment within the same and equivalent scope of the present invention.

[INDUSTRIAL APPLICABILITY]

This invention is the world's first technology developed as a technology for measuring prompt Gamma rays in real time with excellent accuracy and understanding the effects of treatment in BNCT-SPECT, which has not yet been put to practical use due to many restrictions on measuring physical quantities. If an irradiation protocol is established and utilized that delivers a sufficient dose to the tumor while not damaging normal cells, it will be possible to know the effects of treatment in three dimensions in real time and to appropriately control BNCT, thereby greatly improving the reliability of treatment.

Furthermore, it is expected that the BNCT-SPECT of the present invention will greatly contribute to the widespread use of BNCT, when combined with accelerator BNCT using an accelerator neutron source, which is currently becoming more widespread.

[Explanation of symbols]

1       BNCT-SPECT
11      Collimator
11a     Hole formed in collimator
12      Detector (scintillator)
12a     Detector (scintillator) surface
13.     Detector (scintillator) for detecting transmitted gamma rays

**Claims**

1. A measuring device for measuring a therapeutic effect in boron neutron capture therapy, which kills tumor cells by irradiating neutrons to boron that has been accumulated in tumor cells in advance, comprising:

    a collimator having holes of a predetermined shape formed vertically and horizontally at equal intervals;

a detector arranged at the deepest portion of each of the holes for detecting prompt Gamma rays emitted by the irradiation of the boron with the neutrons; and

a calculation processing unit that displays the entire gamma ray intensity distribution three-dimensionally **characterized in**,

when a gamma ray is detected by the detector,

(1) if no gamma ray is detected by other detectors in the vicinity at the same time, a PHS is created based on the single detected gamma ray,

(2) if a gamma ray is detected by other detectors in the vicinity at the same time,

(2-a) if the total number of gamma rays detected is three or more, all signals are discarded and no PHS is created (non-coincidence counting),

(2-b) if the total number of gamma rays detected is two,

(2-b-a) if the sum of the two pulse heights is within a predetermined peak range, the two signals are discarded and the photoelectric peak count corresponding to the predetermined peak is increased by one (coincidence counting);

(2-b-b) if the sum of the two pulse heights is not within a predetermined peak range, the two signals are discarded (non-coincidence counting); and

based on the counts accumulated in each detector within a given time, the overall gamma ray intensity distribution is displayed in three dimensions.

2. The measuring device according to claim 1, wherein

an incidence order determination mechanism that determines an incidence order of the gamma rays into the detector is added to the calculation processing unit, and

the device is configured to perform coincidence counting processing only when the detector into which the $\gamma$-ray first entered is specified.

3. The measurement device according to claim 1 or 2, **characterized in that**;

a detector for detecting transmitted gamma rays and having a size large enough to cover the entire detector is installed on the rear side of the detector, and

the device is configured to perform

when gamma rays are detected simultaneously by the transmitted gamma ray detector and one of the detectors,

(1) when the sum of the pulse heights of the two detected gamma rays is within a predetermined peak range of the PHS, the two detected signals are discarded, and considered to have been detected by the detector, and the photoelectric peak count is increased by one (coincidence counting);

(2) when the sum of the pulse heights is not within a predetermined peak range, the two detected signals are discarded.

4. The measuring device according to claim 1 or 2, wherein the collimator is made of tungsten.

5. The measuring device according to claim 1 or 2, wherein the hole of the collimator is formed in a square tube shape or a cylindrical shape.

6. The measuring device according to claim 5, wherein the collimator hole is formed in a square tube shape with one side of 3 to 5 mm or a cylindrical shape with a diameter of 3 to 5 mm.

7. The measuring device according to claim 5, wherein the length of the hole of the collimator is 20 to 35 cm.

8. The measuring device according to claim 1 or 2, wherein the detector is a semiconductor detector or a scintillator.

9. The measuring device according to claim 8, wherein the detector is a GAGG(Ce) scintillator.

10. A method for measuring a therapeutic effect in boron neutron capture therapy using the measurement device according to claim 1, comprising:

a detection step of detecting a gamma ray, which is emitted promptly by a nuclear reaction with boron due to irradiation with neutrons and then enters a hole in the collimator, by the detector;
a calculation processing step wherein,
if a gamma ray is detected in the detection step,

(1) if no gamma ray is detected by other detectors in the vicinity at the same time, a PHS is created based on the single detected gamma ray,
(2) if a gamma ray is detected by other detectors in the vicinity at the same time,
(2-a) if the total number of gamma rays detected is three or more, all signals are discarded and no PHS is created (non-coincidence counting),
(2-b) if the total number of gamma rays detected is two,
(2-b-a) if the sum of the two pulse heights is within a predetermined peak range, the two signals are discarded and the photoelectric peak count corresponding to the predetermined peak is increased by one (coincidence counting),
(2-b-b) if the sum of the two pulse heights is not within a predetermined peak range, the two signals are discarded (non-coincidence counting); and

a display step of creating and displaying a three-dimensional intensity distribution of the entire gamma rays based on the counts accumulated in each detector within a given time.

11. The measurement method according to claim 10, wherein

a coincidence counting process is performed in the calculation step by using the measurement device according to claim 2;
an order of incidence of the gamma rays on the detector is determined; and then a coincidence counting process is performed only on the gamma ray determined to have been incident first on the detector.

12. The measurement method according to claim 10 or 11, **characterized in that**

a gamma ray is detected by a transmitted gamma ray detector installed on the rear side of the detector, using the measurement device according to claim 3;
when gamma rays are detected simultaneously by the transmitted gamma ray detector and one of the detectors,

(1) when the sum of the pulse heights of the two detected gamma rays is within a predetermined peak range of the PHS, the two detected signals are discarded, considered to have been detected by the detector, and the photoelectric peak count corresponding to the predetermined peak is increased by one (coincidence counting);
(2) if the sum of the two pulse heights is not within a predetermined peak range, the two detected signals are discarded (non-coincidence counting).

13. The measurement method according to claim 10, wherein an accelerator BNCT is used as the neutron irradiation source.

FIG. 1

FIG. 2

FIG. 3

FIG. 4

FIG. 5

1. Coincidence counting method

Use two measurements taken simultaneously

Incident
gamma ray
$(E\gamma = 478 keV)$

$E_{1c}$ 278keV

$E_{2c}$ 200keV

Esum
478keV

The two signals do not form a pulse height distribution
The count of the 478keV photopeak is increased by one

FIG. 6

2. Non-coincidence counting method

Discard both of the two mesured at the same time

Incident
gamma ray
$(E\gamma = 478 keV)$

$E_{1c}$ 278keV

$E_{2c}$ 200keV

No pulse height distribution

FIG. 7

When electrons in this region are measured, it is
determined the element is the first element to be injected

FIG. 8

(a)

(8 × 8 = 64)

Installation of new element (No. 65)

(b)

tumor (r = 1.5cm)

gamma ray
478keV

## INTERNATIONAL SEARCH REPORT

| International application No. |
|---|
| **PCT/JP2023/043056** |

| A. | CLASSIFICATION OF SUBJECT MATTER |
|---|---|

*G01T 1/172*(2006.01)i; *A61N 5/10*(2006.01)i; *G01T 1/17*(2006.01)i; *G01T 1/20*(2006.01)i; *G01T 1/24*(2006.01)i; *G01T 1/161*(2006.01)i
FI:    G01T1/172; G01T1/20 F; G01T1/24; G01T1/17 G; G01T1/17 A; A61N5/10 H; G01T1/161 B

According to International Patent Classification (IPC) or to both national classification and IPC

| B. | FIELDS SEARCHED |
|---|---|

Minimum documentation searched (classification system followed by classification symbols)

G01T1/00; A61N5/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2022-61587 A (CANON MEDICAL SYSTEMS CORP) 19 April 2022 (2022-04-19)<br>entire text, all drawings | 1-13 |
| A | JP 2018-91704 A (SHIMADZU CORPORATION) 14 June 2018 (2018-06-14)<br>entire text, all drawings | 1-13 |
| A | WO 2018/181395 A1 (NIPPON LIGHT METAL COMPANY, LTD.) 04 October 2018 (2018-10-04)<br>entire text, all drawings | 1-13 |

| ☐ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

| Patent document cited in search report | | | Publication date (day/month/year) | Patent family member(s) | | | Publication date (day/month/year) |
|---|---|---|---|---|---|---|---|
| JP | 2022-61587 | A | 19 April 2022 | US | 2022/0104781 | A1 | |
| | | | | entire text, all drawings | | | |
| JP | 2018-91704 | A | 14 June 2018 | US | 2018/0156928 | A1 | |
| | | | | entire text, all drawings | | | |
| | | | | CN | 108152849 | A | |
| WO | 2018/181395 | A1 | 04 October 2018 | US | 2020/0124743 | A1 | |
| | | | | entire text, all drawings | | | |
| | | | | CN | 108990421 | A | |
| | | | | KR 10-2018-0119552 | | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018181395 A